Europäisches Patentamt

European Patent Office

Office européen des brevets

(19)

(11) Veröffentlichungsnummer: **0 017 743**
**B1**

(12) **EUROPÄISCHE PATENTSCHRIFT**

(45) Veröffentlichungstag der Patentschrift:
**23.06.82**

(51) Int. Cl.³: **A 61 F 1/03**

(21) Anmeldenummer: **80101069.5**

(22) Anmeldetag: **04.03.80**

(54) **Gelenkprothese.**

(30) Priorität: **08.03.79 AT 1727/79**

(43) Veröffentlichungstag der Anmeldung:
**29.10.80 Patentblatt 80/22**

(45) Bekanntmachung des Hinweises auf die Patenterteilung:
**23.06.82 Patentblatt 82/25**

(84) Benannte Vertragsstaaten:
**CH DE FR GB IT**

(56) Entgegenhaltungen:
**CH-A-507 704**
**DE-A-2 318 396**
**DE-A1-2 618 763**
**FR-A-2 210 909**
**FR-A1-2 261 743**
**FR-A1-2 295 729**
**FR-A2-2 349 319**
**Z. ORTHOP, Band 115, 1977**
**Stuttgart**
**B. G. WEBER et al. »Aluminiumoxidkeramikkugeln für Hüftendoprothesen nach Baukastenprinzip«**
**Seiten 305 bis 309**

(73) Patentinhaber: **METALLWERK PLANSEE GESELLSCHAFT M.B.H., A-6600 Reutte, Tirol (AT)**

(72) Erfinder: **Schider, Siegfried, Dr., Mittenwaldbahnstrasse 12, A-6600 Reutte-Breitenwang (AT)**

(74) Vertreter: **Lohnert, Wolfgang, Dr., Metallwerk Plansee AG, A-6600 Reutte, Tirol (AT)**

Gelenkprothese

Die Erfindung betrifft eine Gelenkprothese, die einen in einen Knochenkanal einsetzbaren Prothesenschaft und einen am Prothesenschaft befestigbaren Gelenkkopf aus Oxidkeramik aufweist, wobei der Gelenkkopf über ein metallisches Zwischenstück mit dem Prothesenschaft verbunden ist.

Man unterscheidet bei Gelenkprothesen zwischen Partialgelenkprothesen und Totalgelenkprothesen. Bei Partialgelenkprothesen wird nur der Gelenkkopf in Verbindung mit einem Prothesenschaft ersetzt, während bei Totalgelenkprothesen auch die natürliche Gelenkpfanne durch eine Prothese ersetzt wird.

Bei allen Gelenkprothesen tritt an den Berührungsflächen zwischen Gelenkkopf und Gelenkpfanne Reibung auf, die einen Verschleiß dieser Teile bewirkt. Aus diesem Grund müssen die Teile aus Werkstoffen bestehen, die einen geringen Reibungskoeffizienten und eine hohe Verschleißfestigkeit mit geringem Abrieb aufweisen. Gleichzeitig müssen hohe mechanische Festigkeits- und Korrosions-Anforderungen zusammen mit bester Gewebeverträglichkeit sowohl des Prothesenwerkstoffes als auch der entstehenden Abriebprodukte erfüllt sein.

Diese sich zum Teil widersprechenden Anforderungen können, zumindest annäherungsweise, nur durch eine Kombination von verschiedenartigen Werkstoffen erfüllt werden.

Bis heute sind bei Totalprothesen an den reibenden Teilen die Werkstoff-Kombinationen Metall-Metall, Metall-Kunststoff oder Keramik-Keramik eingesetzt oder erprobt worden, wobei der Prothesenschaft in der Regel aus Metall besteht. Als Metalle werden dabei hauptsächlich Co—Cr—Mo- oder Titan-Legierungen verwendet. Der Reibungskoeffizient und der Abrieb bei der Kombination Metall-Metall ist ziemlich groß. Außerdem bewirkt der Abrieb Fremdkörper-Reaktionen mit dem Gewebe und kann so zu Komplikationen führen.

Die Werkstoffkombination Metall-Kunststoff zeigt zwar einen geringen Reibungskoeffizienten, doch gibt es keinen geeigneten Kunststoff, dessen Abrieb keine Fremdkörper-Reaktionen hervorruft und der gleichzeitig ausreichend formstabil und verschleißfest ist. Die Implantationsdauer von Prothesen aus den genannten Werkstoffkombinationen liegt in den besten Fällen bei 5 Jahren.

Die weitaus größte Verschleißbeständigkeit, den geringsten Abrieb und den geringsten Reibungskoeffizienten weist die Kombination Keramik-Keramik auf. Dazu zeigt Keramik eine hervorragende biologische Verträglichkeit, so daß gute Voraussetzungen für eine lange Implantationsdauer bei voller Funktion dieser Prothesen gegeben sind. Bei derartigen Prothesen bestehen Gelenkpfanne und Gelenkkopf aus Oxidkeramik, der Prothesenschaft aus Metall. Die Herstellung von Gelenkkopf und Prothesenschaft in einem Stück aus Oxidkeramik konnte sich nicht durchsetzen, da die Biegebruchfestigkeit der Keramik nur etwa die Hälfte der Biegebruchfestigkeit von Schaftmetallen beträgt und es selbst bei den Metallschäften in Ausnahmefällen zu Schaftbrüchen gekommen ist.

Dadurch ergibt sich das Problem, eine feste verdrehsichere Verbindung zwischen dem Gelenkkopf aus Oxidkeramik und dem Metallschaft herzustellen. Gleichzeitig ergibt sich auch die Forderung nach einer Lösbarkeit dieser Verbindung, um ein Austauschen des Gelenkkopfes zu ermöglichen. Dies ist zum Beispiel in den Fällen notwendig, wo in einem ersten Krankheitsstadium nur eine Teilprothese eingesetzt werden muß, wo also eine implantierte Prothese über über den Gelenkkopf mit der natürlichen Knochenpfanne zusammenwirkt und nach einer gewissen Zeit dann oft die Notwendigkeit der Einsetzung einer Totalprothese besteht. Die Ausgestaltung der Gelenkpfannenprothese zur Erzielung einer ausreichenden mechanischen Festigkeit hat einen kleineren Pfannendurchmesser als bei der natürlichen Pfanne zur Folge. Zur Anpassung des Gelenkkopfes an die künstliche Pfanne wird dann nur der Gelenkkopf ausgetauscht, während der implantierte und im Knochen gut verankerte Prothesenschaft erhalten bleibt. Doch auch die Verschleißteile von Keramik-Keramik-Prothesen müssen bis heute nach spätestens 10—15 Jahren erneuert werden.

In der DE-A-2 451 275 wird eine lösbare Verbindung von Gelenkkopf und Prothesenschaft in der Form vorgeschlagen, daß ein konusförmiger Ansatz am Ende des metallischen Prothesenschaftes in eine entsprechende Ausnehmung in der Gelenkkugel aus Oxidkeramik eingreift und durch Einschlagen eine selbsthemmende Konusverbindung entsteht. Der Nachteil bei dieser Verbindung ist, daß es zu Spannungen in der Keramik kommt, die ein Zerspringen des Gelenkkopfes bewirken können.

In der DE-A-2 618 763 wird eine Verbindung zwischen einem keramischen Gelenkkopf und einem metallischen Prothesenschaft beschrieben, bei der ein Fortsatz des Prothesenschaftes in eine entsprechende Ausnehmung des Gelenkkopfes, die mit einer eingelöteten metallischen Hülse ausgekleidet ist, eingreift. Auch hier besteht der Nachteil, daß der Gelenkkopf durch die Ausnehmung geschwächt und bruchgefährdet ist. Die Gefahr des Zerspringens vom Gelenkkopf ist nicht gelöst, da nach wie vor Metall gegen $Al_2O_3$ geklemmt ist.

Aus der Zeitschrift Z. Orthop., 1977, Band 115, Abb. 5, ist ebenfalls eine lösbare Verbindung zwischen einem $Al_2O_3$-Gelenkkopf und einem metallischen Prothesenschaft beschrieben. Der Gelenkkopf ist dabei auf eine metallische Distanzhülse aufgesteckt, die wiederum mit einem konischen Fortsatz des Prothesenschaftes in Eingriff steht. Auch in diesem Fall besteht der Nachteil, daß der Gelenkkopf durch die Ausnehmung für die Distanzhülse stark geschwächt und damit bruchgefährdet ist.

Des weiteren ist es bekannt, Gelenkkopf und Prothesenschaft miteinander zu verkleben. Neben dem Wegfall der Austauschbarkeit des Gelenkkopfes in der Prothese besteht der Nachteil, daß durch den Klebstoff die Einbringung eines fallweise gewebeunverträglichen Fremdstoffes in das Gewebe erfolgt. Außerdem wird der Klebstoff im Laufe der Zeit abgebaut, so daß sich schließlich die Verbindung lösen kann.

Es ist auch bekannt, eine Schraubverbindung zwischen Gelenkkopf und Prothesenschaft vorzusehen. Dabei wird der Gelenkkopf aus Oxidkeramik mit einem Innengewinde versehen, das mit dem Außengewinde eines Schraubbolzens am Ende des Prothesenschaftes zusammenwirkt. Der Nachteil dabei ist, daß es bei zu starkem Festziehen der Verschraubung wegen der Sprödigkeit der Oxidkeramik zu einem Ausreißen des Gewindes kommen kann. Bei ungenügend fester Verschraubung wiederum kann es durch die Relativbewegung zwischen Gelenkkopf und Gelenkpfanne zu einer Lockerung der Schraubverbindung kommen.

Der Erfindung liegt somit die Aufgabe zugrunde, eine Gelenkprothese der eingangs genannten Art auszubilden, daß der Gelenkkopf aus Oxidkeramik unter Vermeidung der geschilderten Nachteile, wie zum Beispiel der Gefahr des Zerspringens, mit dem Prothesenschaft mechanisch fest verbunden ist und doch die Auswechselbarkeit erhalten bleibt.

Erfindungsgemäß wird die Aufgabe dadurch gelöst, daß das Zwischenstück in etwa vollzylindrisch gestaltet ist und an seinem einen Ende mit dem Gelenkkopf, der keine querschnittsschwächende größere Ausnehmung aufweist über eine Schweißverbindung verbunden ist, während sein anderes Ende über eine Klemm- und/oder Schraubverbindung mit einer entsprechenden Ausnehmung des Prothesenschaftes in Eingriff steht.

Bei einer bevorzugten Ausführungsform der Erfindung besteht der Gelenkkopf aus Aluminium-Oxidkeramik. Das Zwischenstück und der Prothesenschaft bestehen aus Tantal oder Niob, einer Tantal- oder Nioblegierung oder aus einem mit Tantal oder Niob beschichteten Grundkörper. Die Verbindung Zwischenstück mit Prothesenschaft ist eine selbsthemmende Konusverbindung oder eine gesicherte Schraubverbindung.

Die Schweißverbindung ist bevorzugt eine Druckdiffusions-Schweißverbindung. Die durch die Druckdiffusions-Schweißverbindung zu verbindenden Berührungsflächen des Gelenkkopfes und des Zwischenstückes sind vorzugsweise Kegelmantelflächen. Die Anwendung der Druckdiffusions-Schweißtechnik bewirkt unter Verwendung einer Niob-Zwischenschicht eine hervorragende, ausreichend feste Verbindung zwischen dem Gelenkkopf aus Aluminium-Oxidkeramik und dem Zwischenstück aus Tantal, ohne eine Verschlechterung der mechanischen Eigenschaften der zu verbindenden Materialien zu bewirken.

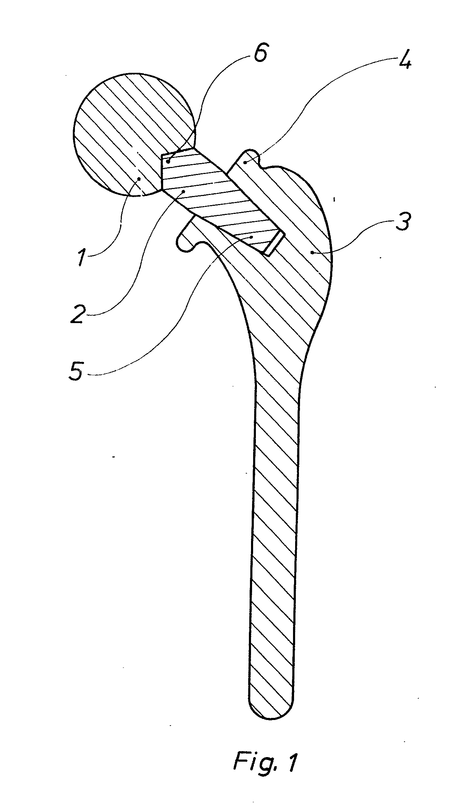

Eine Ausführungsform der Erfindung als Hüftgelenksprothese wird an Hand der Fig. 1 beschrieben, die die Ansicht einer erfindungsgemäßen Hüftprothese im Schnitt zeigt.

Der Gelenkkopf —1— aus Aluminium-Oxidkeramik ist an seinem Umfang mit einer kegelförmigen Ausnehmung versehen, in die das entsprechend ausgebildete Ende —6— des Zwischenstückes —2— aus Tantal eingreift. Die feste Verbindung dieser zwei Teile erfolgt mittels Druckdiffusions-Schweißung unter Verwendung einer Niob-Zwischenschicht. Das dem Gelenkkopf —1— abgewandte Ende —5— des Zwischenstückes —2— läuft kegelstumpfförmig zu und greift in eine entsprechend ausgebildete Ausnehmung am oberen Ende des Prothesenschaftes —3— ein. Durch leichtes Einschlagen des Gelenkkopfes —1— mit dem Zwischenstück —2— in diese Ausnehmung wird eine mechanisch feste, jedoch wieder lösbare, selbsthemmende Konusverbindung mit dem Prothesenschaft —3— erreicht. Der Prothesenschaft —3— aus Tantal ist an seinem oberen Ende mit einem Kragen —4— versehen, der zur Auflage und Abstützung der Prothese am Stumpf des Oberschenkelknochens dient, um unter Belastung ein Absinken der Prothese im Femurschaft mit möglicher Spaltung des Knochens zu verhindern. Der Vorteil der erfindungsgemäßen Gelenkprothese liegt darin, eine mechanisch feste, lösbare Verbindung zwischen Gelenkkopf aus Oxidkeramik und metallischem Prothesenschaft herzustellen, ohne daß es zu einem Zerspringen des Gelenkkopfes oder einer unbeabsichtigten Lockerung der Verbindung kommen kann.

Bei den bekannten Problemen mit gebräuchlichen Metall-Keramik-Verbindungen, auf Grund derer auch auf dem Sektor Prothesen Mißerfolge in der Vergangenheit nicht ausgeblieben sind, stellt die Anwendung der Druckdiffusions-Schweißtechnik eine entscheidende Hilfestellung für vorliegende Erfindung dar. Diese Technik führt zu einer ausreichend festen Verbindung zwischen Metall und Keramik, ohne daß die Verwendung gewebeunverträglicher oder korrosionsfördernder, haftvermittelnder Zusatzwerkstoffe erforderlich ist.

**Patentansprüche**

1. Gelenkprothese, die einen in einen Knochenkanal einsetzbaren, metallischen Prothesenschaft (3) und einen am Prothesenschaft befestigten Gelenkkopf (1) aus Oxid-Kermaik aufweist, wobei der Gelenkkopf über ein metallisches Zwischenstück (2) mit dem Prothesenschaft verbunden ist, dadurch gekennzeichnet, daß das Zwischenstück (2) in etwa vollzylindrisch gestaltet ist und an seinem einen Ende (6) mit dem Gelenkkopf (1), der keine querschnittsschwächende größere Ausnehmung aufweist, über eine Schweißverbindung verbunden ist, während sein anderes Ende (5) über eine Klemm- und/oder Schraubverbindung mit einer entsprechenden Ausnehmung des Prothesenschaftes (3) in Eingriff steht.

2. Gelenkprothese nach Anspruch 1, dadurch gekennzeichnet, daß der Gelenkkopf (1) aus Aluminium-Oxidkeramik besteht.

3. Gelenkprothese nach Anspruch 1 oder 2, dadurch gekennzeichnet, daß das Zwischenstück (2) und der Prothesenschaft (3) aus Tantal oder Niob, einer Tantal- oder Niob-Legierung oder aus einem mit Tantal oder Niob beschichteten Grundkörper bestehen.

4. Gelenkprothese nach einem der Ansprüche 1 bis 3, dadurch gekennzeichnet, daß die Verbindung Zwischenstück (2) — Prothesenschaft (3) eine selbsthemmende Konusverbindung ist.

5. Gelenkprothese nach einem der Ansprüche 1 bis 3, dadurch gekennzeichnet, daß die Verbindung Zwischenstück (2) — Prothesenschaft (3) eine gesicherte Schraubverbindung ist.

6. Gelenkprothese nach einem der Ansprüche 1 bis 5, dadurch gekennzeichnet, daß die Schweißverbindung eine Druckdiffusions-Schweißverbindung ist.

7. Gelenkprothese nach Anspruch 6, dadurch gekennzeichnet, daß die mittels Druckdiffusions-Schweißung verbundenen Berührungsflächen des Gelenkkopfes (1) und des Zwischenstückes (2) Kegelmantelflächen sind.

## Claims

1. Joint prothesis, comprising a metallic prothesis shaft (3) implantable in a bone channel and a joint head (1) of oxide-ceramics, mountable on the prothesis shaft, wherein the joint head is connected to the prothesis shaft by means of a metallic connecting piece (2), characterized in, that the connecting piece (2) is in its shape approximately solid cylindrical and is connected at its one end (6) by means of a welding joint to the joint head comprising no substantial size diminishing recess, whereas its other end (5) is connected to the prothesis shaft (3) by a clamping and/or a screw joint.

2. Joint prothesis of claim 1, characterized in, that the joint head (1) consists of alumina.

3. Joint prothesis of claims 1 or 2, characterized in, that the connecting piece (2) and the prothesis shaft (3) consist of tantalum or columbium, an alloy thereof or of a body coated with tantalum or columbium.

4. Joint prothesis of claims 1 to 3, characterized in, that the joint between the connecting piece (2) and the prothesis shaft (3) is a self stopping cone joint.

5. Joint prothesis of claims 1 to 3, characterized in, that the joint between the connecting piece (2) and the prothesis shaft (3) is a secured screw joint.

6. Joint prothesis of claims 1 to 5, characterized in, that the welding joint is a pressure diffusion welding joint.

7. Joint prothesis of claim 6, characterized in, that the contact surfaces of the joint head (1) and the connecting piece (2), connected by means of pressure diffusion welding, are conical.

## Revendications

1. Prothèse d'articulation du type comportant une tige de prothèse (3) pouvant être logée dans un canal dós et une tête d'articulation (1) en céramique oxydée pouvant être fixée sur la tige de prothèse, la tête d'articulation étant couplée à la tige de prothèse par l'intermédiaire d'une pièce intermédiaire métallique (2), caractérisée en ce que la pièce intermédiaire (2) a sensiblement la forme d'un cylindre plein et elle est couplée par une soudure à une extrémité (6) avec la tête d'articulation (1) qui ne comporte pas d'évidement trop important affaiblissant la section transversale, tandis que son autre extrémité (5) coopère, par l'intermédiaire d'un couplage par serrage et/ou vissage, avec un évidement correspondant de la tige de prothèse (3).

2. Prothèse d'articulation suivant la revendication 1, caractérisée en ce que la tête d'articulation (1) est constituée par de la céramique aluminée.

3. Prothèse d'articulation suivant l'une des revendications 1 ou 2, caractérisée en ce que la pièce intermédiaire (2) et la tige de prothèse (3) sont constituées par du tantale ou du niobium, un alliage de tantale ou de niobium ou par un corps de base recouvert de tantale ou de niobium.

4. Prothèse d'articulation suivant l'une des revendications 1 à 3, caractérisée en ce que le couplage de la pièce intermédiaire (2) et de la tige de prothèse (3) est un couplage à cône auto-bloquant.

5. Prothèse d'articulation suivant l'une des revendications 1 à 3, caractérisée en ce que le couplage de la pièce intermédiaire (2) et de la tige de prothèse (3) est un couplage à vis bloqué.

6. Prothèse d'articulation suivant l'une des revendications 1 à 5, caractérisée en ce que la soudure est une soudure par diffusion sous pression.

7. Prothèse d'articulation suivant la revendication 6, caractérisée en ce que les surfaces de contact, réunies par une soudure par diffusion sous pression, de la tête d'articulation (1) et de la pièce intermédiaire (2) sont des surfaces d'aire latérale de cône.

Fig. 1